# EUROPEAN PATENT SPECIFICATION

(11) **EP 0 650 709 B1**
(45) Date of publication and mention of the grant of the patent: **02.09.1998**
(21) Application number: 94307959.0
(22) Date of filing: 28.10.1994
(51) Int. Cl.: A61F 5/448

(54) **Ostomy coupling**
Ostomiekupplung
Accouplement d'ostomie

(30) Priority: 28.10.1993 GB 9322235
(43) Date of publication of application: 03.05.1995
(73) Proprietor: E.R. Squibb & Sons, Inc., Princeton, N.J. 08540-4000 (US)
(72) Inventor: Steer, Peter Leslie, East Grinstead, Sussex (GB); Bannister, Graham David, Lindfield, West Sussex RH16 2ND (GB)
(74) Representative: Holmes, Miles Keeton

(56) References cited:
- EP-A- 0 286 501
- EP-A- 0 463 359
- EP-A- 0 489 017
- EP-A- 0 509 764
- EP-A- 0 519 586
- EP-A- 0 629 388
- US-A- 5 167 651

## Description

This invention relates to an ostomy coupling. Such couplings are used to attach a bag for receiving discharged material to a medical grade adhesive pad which is attached to the wearer's peristomal skin surface.

Many designs of such couplings have been made. A large number of these have been unduly complex and expensive. A few have been simple in design and fewer still have been simple and effective.

European Patent Application No. 286501A shows an ostomy coupling system in which a rotable lock ring can assume two positions, in one of which two coupling rings are connected and in the other of which they can be disconnected.

European Patent Application No. 519586 shows a pair of coupling rings, one of channel form, into which is pushed a non-compressible bulbous insert having a pivot stem and forming part of the other coupling ring.

European Patent Application No. 489017 shows a two piece coupling system of the general kind shown in EP 519586 or EP 509764. The '017 system involves two inter-engaging parts of particular shape and design. The intention is to obtain a remarkable imperviousness between the closure elements of the male and female coupling elements.

European Patent Application No. 629388 (published 21/12/94 claiming a priority of 03/06/93) shows a two-piece ostomy appliance having a flip-over ring structure (in principle but not in detail similar to US Patent Application No. 5167651) mounted on a faceplate.

Among the characteristics which are desirable in an ostomy coupling are:-
ease of manipulation, particularly for elderly or infirm users;
the mutual interconnection between the first and second coupling elements, and hence between the bag and the medical grade adhesive pad, must be reliable but not require high forces;
the overall appliance must have a small thickness and hence minimise the possible embarrassment due to the appliance projecting so much that it is visible under clothing; in other words the "outstand" must be minimised; and
manipulation of the coupling elements must be such that it is easy to disconnect a bag when it is full and to replace it a fresh, empty bag.

In the Applicants' opinion, no existing design of ostomy appliance is fully satisfactory under all these headings. It is an aim of the present invention to provide a particularly simple yet effective ostomy coupling. Also, ostomy systems featuring a "locking" mechanism generally employ a third component. It is believed this application embodies the first practical "two piece" locking system in an ostomy coupling.

According to a principal aspect of the present invention, there is provided an ostomy coupling an ostomy coupling which includes a body side coupling element comprising a flange from which extends a substantially cylindrical rib or wall the flange having a medical grade adhesive pad attached thereto, the rib having a flexible and deflectable seal strip located radially inwardly thereof, the bag side coupling element having a base wall attached to a substantially annular wall positioned for engagement with, and so as to be encircled by, the seal strip on the body side coupling element, the bag side coupling element also having an annular outer wall of resilient stretchable synthetic plastics material which encircles the base wall and is joined to the base wall by an integral annular plastics hinge whereby in use the said outer wall acts as a flip-over ring and can be snapped between two limiting positions by direct finger action, the wall in its first or open position extending generally outwardly of the base wall and in its second or closed position being located adjacent to both the flange and the wall of the body side coupling element.

To the best of the Inventor's knowledge and belief, an ostomy coupling according to this invention is the first ostomy coupling, not involving any locking ring or similar mechanism such as is seen in European Patents or Applications Nos. 255310, 286501, 461007 and 482104, and being of simple design basically composing only two parts, in which a positive, effective and reliable locking together of the two coupling parts is achieved. The coupling according to U.K. Patent No. 1571657 involved a snap-in push fit to secure the two coupling parts, but the coupling according to the present invention provides a superior permanence of attachment, by virtue of the locking engagement of part of the flip-over ring with the confronting body-side coupling. To achieve this with only two simple moulded components is thought to be a considerable advance in a well-populated art.

A suggestion has been made, in U.S. Patent No. 5,167,651 of Leise and Lavender, that a simple two-piece ostomy appliance should include a dual-state, bistable coupling ring directly attached to the wall of an ostomy pouch. In this arrangement, the ring is formed of relatively stiff but flexible plastic material and is capable of assuming two stable states or conditions. In one such state, its normal one, the ring is substantially untensioned and its configuration is frusto-conical; in its other state, the direction of the conical taper is reversed or inverted and the ring again assumes a stable but now tensioned condition. When in its normal state, the ring has an inside diameter substantially smaller than in its inverted state, so that in mating such rings together, a user may enlarge the opening to facilitate joining the parts together and may then return the bistable ring to its normal state to insure a secure, fluid-tight seal between the parts. Flip-over, or bistable, rings may be made of plastics material. Such rings are well known, and have been employed for many years on paint tin lids and plastics containers such as those in which charcoal briquettes are sold.

With an arrangement as particularly disclosed and illustrated in the present application, by finger leverage, the outer wall of the second coupling element can be flipped over, or "snapped" between respective closed and open positions. In its open position, the two coupling elements can readily be separated.

For this purpose, if desired, one or more gripping tabs can be placed on each of one or both elements. However, in the closed position of the wall, the preferred construction is such that there is a detent or locking relationship existing between an overhang on the radially outer rim of the cylindrical rib and a step on the radially inner surface of the outer wall of the second coupling element.

According to another aspect of the present invention, there is provided a ring-like coupling element of an ostomy coupling having a channel shape in cross section, defined by radially inner and outer walls and a floor, the element being characterised in that (i) the floor of the element has a plurality of depression therein adjacent to the inner wall, the depressions being equally spaced around the channel, and (ii) the radially outer wall has a plurality of inwardly-projecting shelf-like roof portions, equally spaced around the channel and extending partly across the channel from that part of the outer wall furthest from the channel floor.

With this arrangement, the security of attachment of the two coupling parts is enhanced by the overlap of the roof portions and the other coupling element; that is to say, the overlap of the roof portions tends to prevent the second (e.g. bag side) moving out of the channel in the axial direction of the coupling. Also, the manufacture of the body side coupling element using injection moulding techniquies is facilitated.

The roof portions are preferably shaped so that they have a substantially flat surface, substantially parallel to the channel floor, and a curved shape on their inner and upper surfaces. This latter shape renders the two coupling elements relatively easy to push (when they are being coupled together) into a mutually engaged position.

In an arrangement according to this aspect of the invention, a "flip over" action of the outer wall as described above is included, by providing a plastics hinge connecting the said outer wall to the remainder of the channel-shape element.

The invention will be better understood from the following illustrative and non-limiting particular description of embodiments thereof, given with reference to the accompanying illustrative drawings, in which like parts are denoted by like reference numerals and in which:-
Figure 1 is a front view of a first coupling element according to one embodiment of the invention;
Figure 2 is a cross section taken in an axial plane of the coupling on the line A-A of Figure 1;
Figure 3 is a similar cross sectional view but illustrating one embodiment of a second coupling element according to the invention, and Figure 3A is a diagrammatic plan view of this element;
Figures 4 and 5 show mutually interengaged first and second coupling elements in respectively their closed and open positions;
Figures 6 and 7 illustrate, in axial cross-section, a second embodiment of the invention, Figure 6 showing first and second coupling elements mutually locked together, and Figure 7 shows the same two coupling elements in unlocked condition, the "flip over" or "snap-over" wall being seen in its initial position at the upper part of Figure 7;
Figures 8-10 illustrate a bag-side coupling element according to one example of a further embodiment of the invention, this element being suitable for co-operation with a body-side coupling element as illustrated in Figure 11 herein. Figure 8 is a plan view of a bag-side coupling element, and Figure 8A shows detail of a belt tab thereon;
Figure 9 is a cross-sectional view on the line II-II of Figure 8, of the bag side coupling element illustrated in Figure 8;
Figure 10 is an enlargement of part of Figure 8 illustrating the depressions and the roof portions; and
Figure 11 is a cross-sectional view of a co-operating ostomy (male) coupling element, intended to inter-engage with the coupling element shown in Figures 8-10.

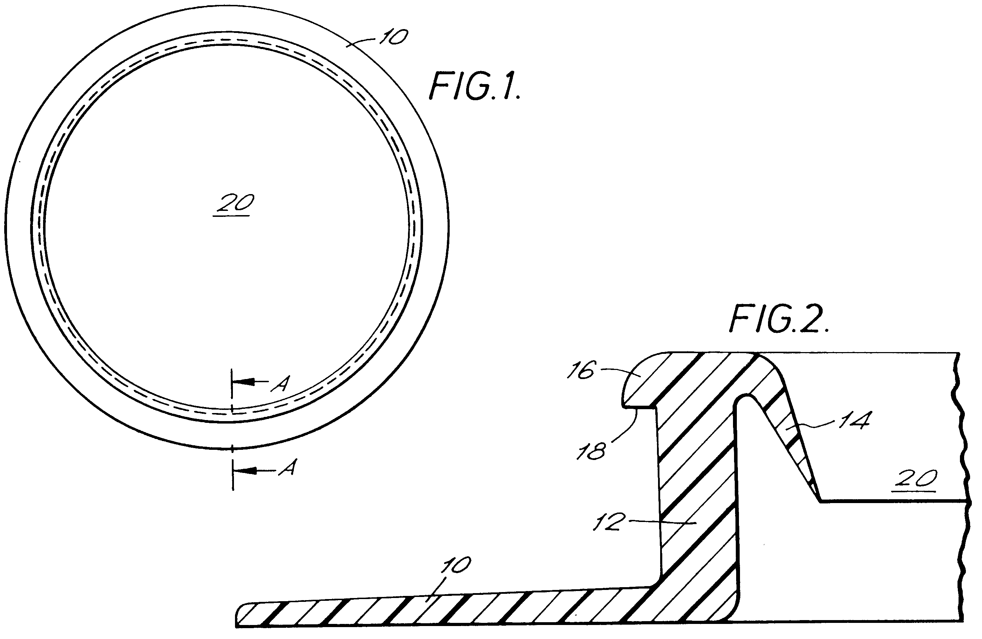

Referring to Figures 1 and 2, the first coupling element shown therein is circular in form and comprises a flange 10 and an approximately cylindrical rib 12. While in the present particular description of the invention, reference is made to a cylindrical rib and approximately circular coupling elements, it will be appreciated that satisfactory ostomy coupling elements could be made which are not circular or cylindrical. For example, an ostomy coupling appliance could be made with an oval stomal orifice, or with stomal orifice of square or rectangular shape having rounded corners. The disclosure in this patent application are accordingly not to be regarded as limited to geometrically circular constructions of ostomy appliance.

The flange 10 may have an outside diameter (O.D.) of about 94 mm. and the rib 12 may have an I.D. of about 77 mm. in the case of an ostomy appliance of nominal 70 mm. size; and the flange 10 may have an O.D. of about 124 mm. and the rib 12 may have an I.D. of about 107 mm. in the case of an ostomy appliance of nominal 100 mm. size. Appliances of other sizes may of course be made utilizing the features of this invention.

The rib 12 has extending therefrom radially inwardly a flexible and deflectable seal strip 14, of tapering form as illustrated, and the radially outer portion of the rib has a rim portion 16 which has a flat surface 18. The stomal orifice of the coupling element is seen at 20. As illustrated, the flange 10 tapers to a lesser thickness towards its outer rim, but this flange alternatively may be of uniform thickness, if desired.

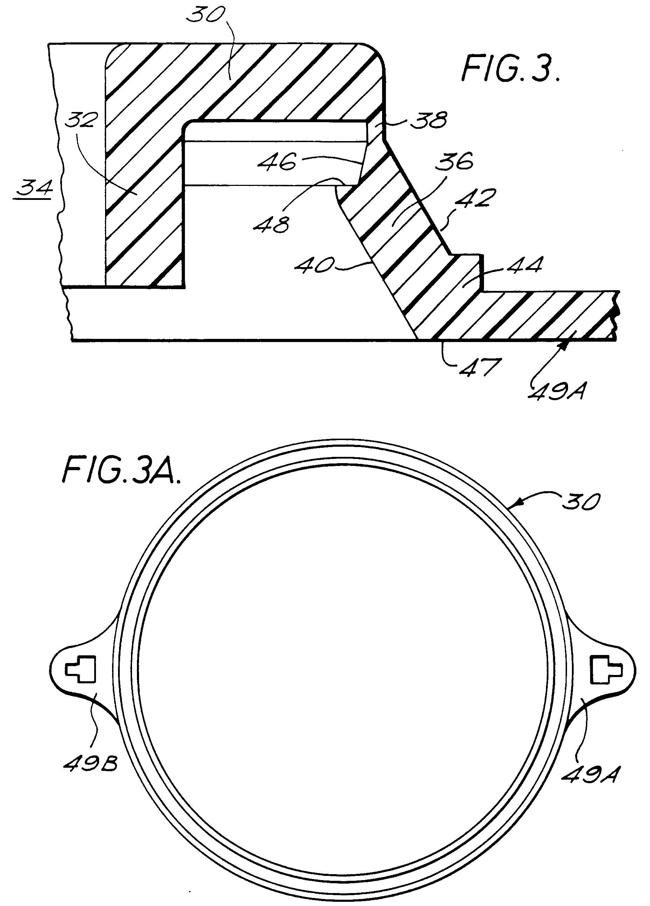

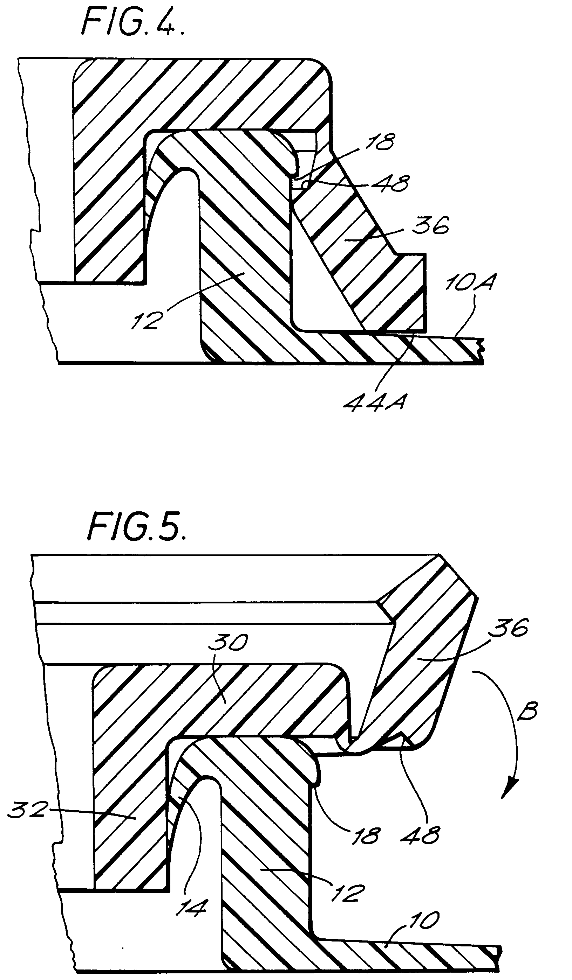

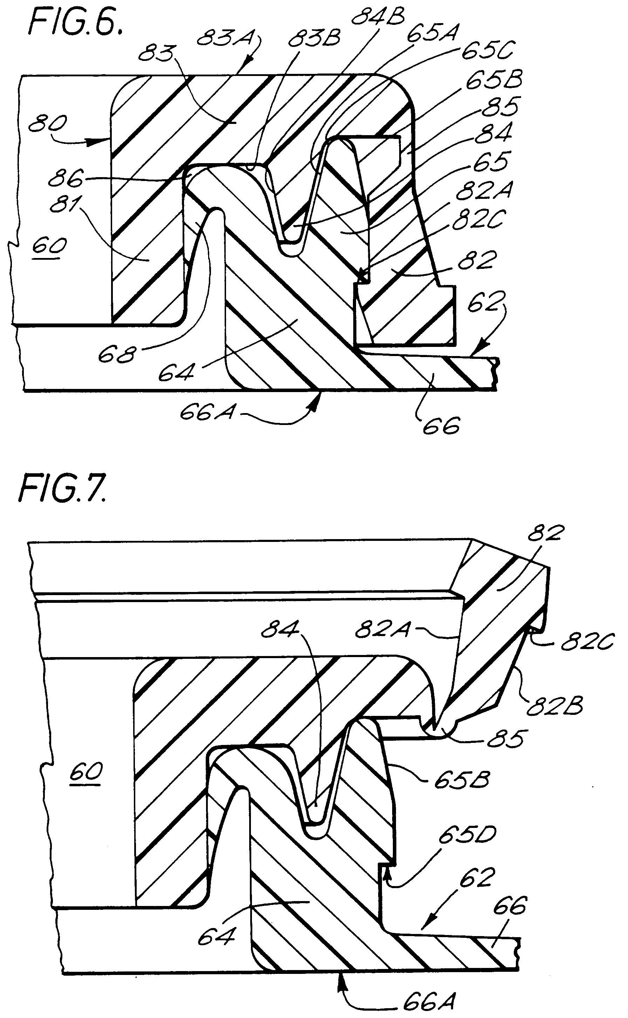

Referring now to Figure 3, the second coupling element is of annular form and comprises a base 30, integral with a generally cylindrical inner wall 32 which defines stomal orifice 34 and also has an outer wall 36 which is connected to the base 30 by an integral plastics hinge 38. The plastics hinge 38 forms the thinnest portion of the outer wall 36. The outer wall 36, as seen in its locking or detent position (i.e. Figs. 3 and 4), is defined by sloping radially inner and outer surfaces 40, 42 and there is a rim portion 44 located at the end of the wall 36 remote from the hinge 38. The wall 36, however need not have parallel inner and outer surfaces as seen in cross-section, and could be of any suitable cross-sectional shape. The thickness of the hinge is chosen according to the selected plastics material, taking account of conventional practice in the design of a plastics hinge. It may be approximately 7% to 10% of the width (in a radial direction of the coupling) of the channel. The width of the channel may be about 3.8 mm. and the thickness of wall 32 may be about 1.5 mm. Adjacent to the hinge 38, the wall 36 has an inwardly sloping surface 46 and a step 48 whose function will be apparent from the following description. The surface 46, as seen in its locking position, may be disposed at an angle of about 13-17 degrees to the axis of revolution of the illustrated coupling, but other shapes of wall may be employed, see, for example, Figures 6 and 7 herein. The angles of the surfaces 40 and 42 to the said axis may be, for example, about 25 to 35, or more preferably, 30 degrees. The wall 36 seen in Figure 3 has integral therewith, or attached thereto, at two locations, tabs 49A and 49B which are preferably disposed at opposite ends of a diameter of the coupling element 30. The form of these two tabs is best seen from Figure 3A which is a diagrammatic plan view of the coupling element 30, detail of the precise structure of the channel having been omitted since it is readily understood from Figures 3-5. Each belt tab 49A or 49B comprises a thin flat tab of plastics material having therein a "T"-shaped hole. The hole, however, may be of any convenient shape. The tabs need not be two in number nor need they be located at opposite ends of a diameter; they may be located where desired on the periphery of the wall 36 so that they can be conveniently gripped to facilitate the "snap-over" action.

The co-operation between the first and second coupling elements will be understood by viewing Figures 4 and 5. Starting from the unlocked position, Figure 5, the outer wall 36 can be snapped by finger action in the direction of the arrow B to take up the position illustrated in Figure 4. In that position, the surface 44A of the portion 44 is close to the upper surface 10A of the flange 10 and the surface of the first coupling element rib 12 overhangs the surface 48 of the outer wall 36. As explained, the material of the second coupling element is to some extent elastic, and hence the natural resilience of the encircling outer wall 36 when in its Figure 4 detent or locking position will tend to prevent any radially outer movement of the wall 36. Any outward movement of the wall 36 would require it to deform or stretch to some extent. Consequently, due to the overlap of surface 18 and the positioning relative thereto of surface 48, separation of the coupling parts is normally prevented. However, a certain amount of relative axial movement is possible, through the distance indicated "C" in Figure 4, this spacing being provided as a tolerance to facilitate moulding. Consequently if a wearer wishes to separate the two coupling elements, he or she merely pulls one or both of the belt tabs 49A, 49B. It is preferred to pull just one belt tab, as this starts a "peeling action" kind of separation between the wall 36 and the coupling element 12 at one peripheral position. Once this peel is started, the resilience and deformability of the wall 36 enables it to be snapped over and open, and the two coupling elements can then be readily separated.

The extent of overhang of the surface 18 seen in Figure 2 is approximately 40 to 50% of the thickness of the rib 12 in the Figure 2 embodiment of the invention and approximately 10% in the Figures 4 and 5 embodiment. However, this ratio may be changed within wide limits, for example the overhang (width) of the surface 18 is preferably at least 10% and may have a value up to about ²/₃ of the thickness of the rib 18. Increasing this overhang obtains an even more secure coupling between the coupling elements.

Referring now to Figures 6 and 7, which show another embodiment of the invention, this embodies an anti-tilt feature of construction, combined with the feature of a "snap-over" wall 82 which enables secure locking together of the two coupling elements. It will be appreciated that Figures 6 and 7 each show a section at one end of a diameter; each coupling element is of closed loop form and surrounds a stomal orifice 60. Coupling element 62 is a body side element and has an annular wall 64 and an annular flange 66. A medical grade adhesive pad, not shown, is attached in any suitable way to the surface 66A of the flange 66. This pad, in use, adheres to the wearer's peristomal area. The wall 64 and the flange 66 are preferably made integral with each other and the coupling element may be moulded from synthetic plastics material. An annular flexible and deflectible sealing strip 68 is integral with and extends from the periphery of the wall 64, in the manner shown. This strip is provided to obtain good sealing between the coupling parts. In this connection, the attention of the reader is drawn to U.K. Patent No. 1,568,860.

Also extending from the wall 64, is an annular rib 65 having tapering surfaces 65A and 65B joined by a rounded surface 65C. The surface 65B terminates in a peripheral rim 65D (Fig. 7) which stands slightly proud of the external surface of the wall 64. The purpose of the described formations can best be understood by referring to Figure 6, from which it is seen that the rim 65D co-operates with a part of the other coupling element 80 to hold the two parts together. The seal strip 68 bears against a wall of the element 80.

The coupling comprises a second coupling element 80 which is basically an annular and generally U-shaped channel. In Figures 6 and 7 it is seen in cross-section. The coupling element 80 has an inner wall 81, an outer wall 82, and a base 83. An ostomy bag is attached in any convenient way to the surface 83A of the base 83. On the opposite side of the base from the surface 83A, there is provided a rib 84 which has tapering surfaces 84A and 84B joined by a rounded surface. Taking the depth of the channel measured at wall 81 as the distance d, the rib 84 extends from the base 83 a distance approximately equal to half the depth d. The wall 84B of the rib 84 merges into a wall 83B which forms part of the base 83. The outer wall 82 of the channel-shaped element is joined by an integral plastics hinge 85 to the base 83. The wall 80 is of tapering shape as seen in section, and has surfaces 82A and 82B. The surface 82B has a step 82C thereon which in the closed (locking) position of the wall 82 engages the step 65D (Fig. 7) on the wall 64, so holding the two coupling elements securely together. The width (radial extent) of the steps 82C and 65D can be varied as desired, bearing in mind that the widths should not be reduced to such an extent that secure locking is prejudiced. To unlock, as in the previously-described embodiment of the invention, one pulls on one of the belt tabs to "snap over" the wall 82 to move it to the position seen in Figure 7.

The inter-engagement between the rib 84 and the counterpart recess in the coupling element 64 together with the inter-engagement between the seal strip 68 and the recess 86 (Figure 6) and the engagement at 65D, 82C as described gives rise to a very secure inter-engagement which is highly resistant to and effectively prevents tilting of one coupling element relative to the other. This advantage is obtained without any increase in the expense or difficulty of manufacture of the parts, and leaves unaffected all the advantageous features of an ostomy coupling of this general type.

Modifications may be made without departing from the invention. For example, as already indicated, the member 62 may be the bag side member and the member 80 may be the body side member. Different specific shapes may be employed for the flexible seal strip 68 and the ribs 65 and 84. While it is advantageous to have the rounded formation 65C and the peak of the rib 84 rounded, this is not essential and the tapered walls may join at an apex (as seen in cross-section) if desired.

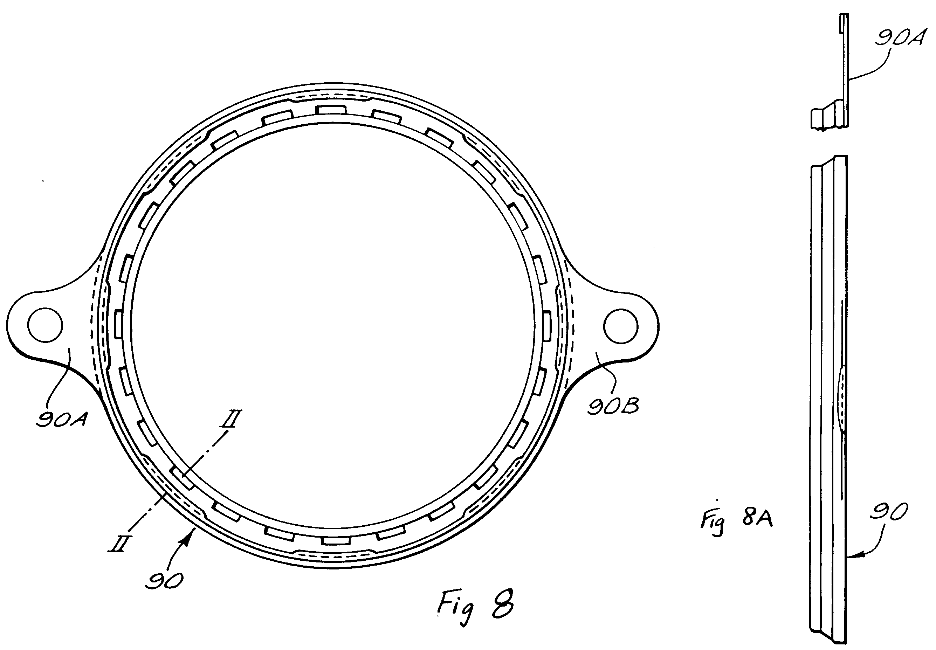

Referring now to Figures 8-11, a bag side coupling element according to a further embodiment of the invention is illustrated in Figures 8-10. It comprises a channel section element 90 of circular form as seen in Figure 1. However, those skilled in the art will appreciate that ostomy couplings of other than circular form, e.g. oval, are possible and the present invention is not considered to be limited to ostomy coupling elements which are geometrically circular. The female ostomy coupling element of Figures 8-10 may be normally made of a synthetic plastics material such as EVA. EVA Grade UL 00209 is one material, available from Esso, which is suitable. The male element of Figure 11 may be made of low density polyethylene.

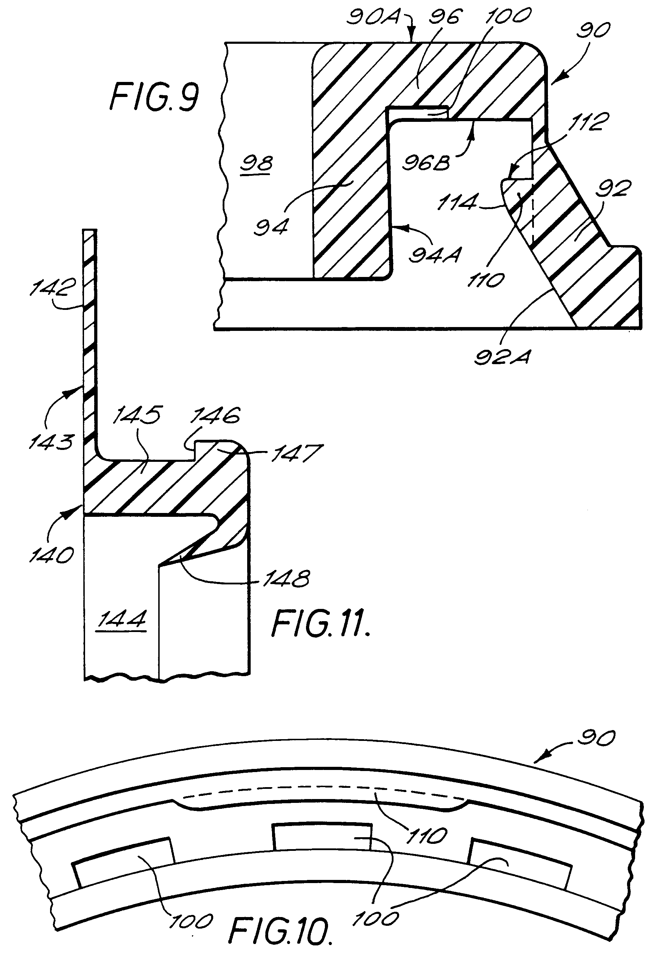

The element 90 as seen in Figure 8 has tabs 90A, 90B. The tabs 90A and 90B are provided to facilitate attachment of a belt, and either may be used to afford a grasping point whereby the wearer may separate the two coupling elements by "peeling" and then "snapping over" the wall as described above. The coupling element 90 has a radially outer wall 92 and a radially inner wall 94. The wall 94 surrounds a stomal orifice 98 at the centre of the coupling. The generally ring-like or annular channel-like element 90 has a floor 96 which joins the radially inner and outer walls 92, 94, this floor having a surface 96A to which may be attached, e.g. by adhesive, heat welding, RF plastics welding, or other suitable method, an ostomy bag provided with a suitable hole in one of its walls to serve as an entry for discharges excreted by the wearer. This entry hole (not shown) of the bag is aligned with the stomal orifice 98 as is conventional in ostomy bags. The floor 96 has a surface 96B at the base of the channel, and, into this floor, there are provided a plurality of recesses 100. These recesses 100 in their presently preferred form are relatively shallow, flat bottomed, and are equally spaced peripherally around the coupling element. As seen best in Figure 10, the recesses 100 have a shape which is approximately rectangular. In a preferred embodiment of this aspect of the invention, there are 24 such recesses, spaced around the ring at 15° centres. The depth of each such recess is approximately 0.01 inch, i.e. approximately .254 millimetres. The remainder of the surface 96B at the base of the channel is substantially flat.

The radially outer wall 92 is joined to the base 96 by an integral plastics hinge 95 and is provided with a radially inwardly projecting roof-like portion 110 whose underside 112 is substantially flat and extends substantially in a plane parallel to the planes of the respective surfaces 96A and 96B when the wall 92 is in its locking position. In its unlocked position, permitting the two coupling elements to be separated, its takes up a position similar to wall 82 as seen in Figure 7, or wall 36 as seen in Figure 5. The portion 110 extends inwardly a distance such that it covers from about 11 to about 18%, and preferably about 15%, of the width of the channel. The roof-like portion 110 (in its closed position as seen in Fig. 9) has its upper and inner surface defined by a shallow curve 114 that joins the surfaces 112 and 92A.

The radially inner wall 94 is, as seen in Figure 9, of greater height than distance between the surfaces 112 and 96B.

The respective roof portions 110 are equally spaced around the inner periphery of the wall 92, and are preferably located at 45° centres. The extent in a peripheral sense of each roof portion 110 is such that it subtends, at the centre in the case of a geometrically circular ostomy coupling element, an angle between about 18 and 22° and preferably about 20°. The number, positioning, and angular extent of the roof portions 110 may be varied. The roof portions 110 may extend inwardly of the wall 92 by an amount equal to about one fifth to one seventh, preferably one sixth, of the width of the channel measured in a radial direction. It has been found that this amount of inward extent, taken together with the positioning, number and arrangement of the roof portions, gives an ostomy coupling in which the security of attachment of the two coupling elements is enhanced, but the ease of separation of the two coupling elements by the "peel and then snap" method described in detail above is such that the separation can be readily achieved by virtually all ostomy coupling wearers.

Figure 11 illustrates, in partial cross-section, an ostomy coupling element 140, which is also of ring-like or annular form and intended to interfit with the element 90. In the preferred embodiment of the present invention, the element 140 would be the body side coupling element. It comprises a radially extending substantially flat flange 142 and a peripherally extending rib member 145 formed for mutual inter-engagement with coupling element 90. The flange 142 has a surface 143 to which is secured, in any suitable manner, a pad (not shown) of medical grade adhesive material. Suitable adhesive materials for this purpose and for a like purpose in other embodiments of this invention include, for example, those described by Chen in U.S. Patent 3,339,546; by Chen et al. in U.S. Patent No. 4,192,785; by Pawelchak et al. in U.S. Patent No. 4,393080; and by Doyle et al. in U.S. Patent No. 4,551,490. A particularly suitable material is that sold under the Registered Trade Mark STOMAHESIVE by Bristol-Myers Squibb of Ickenham, Middlesex, England. As is conventional in ostomy couplings, provision may be made on the free surface of the medical grade adhesive pad for markings directed to assisting the user to cut out a central portion of the pad, in registry with the stomal orifice 144. The rib member 145 has a radially outer rim 147 and a radially inner flexible deflectible seal strip 148. The purpose of the strip 148 is to assure good sealing between the two coupling elements when they are inter-engaged. The rim 147 has a step 146, located substantially parallel to the plane of the flange 142. Of course alternatively the surface may be at an angle, preferably a small angle, to the plane of the flange 142. When the two coupling parts are connected together, and the wall 92 is in its snapped over or closed position (similar to Fig. 6) there is a secure locking engagement between surfaces 146 and 112 which holds the two coupling elements firmly together. The seal strip 148 is deflected slightly and bears resiliently against the surface 94A of the wall 94, so providing a good seal and taking up any minor tolerance variations which may have arisen in manufacture.

In the above description, reference has been made to the surface 112 being located in a plane substantially parallel to the planes of the surfaces 96A and 96B. It is not essential to the present invention that the surface 112 should be planar nor that it should be precisely parallel to the planar surfaces 96A and 96B.

While the coupling element 90 disclosed herein has been described as having 24 recesses 100, at equal angular spacings, a different number of such recesses could be employed if desired.

Those skilled in the art of design of ostomy couplings will realise that certain changes and modifications may be made, without departing from the present invention. For example, as mentioned, coupling elements need not be precisely circular so long as they are of complementary shape and can be joined together so as to provide a well-sealed coupling. Other variations will occur to a man skilled in the art.

## Claims

1. An ostomy coupling which includes a body side coupling element comprising a flange (10,66,142) from which extends a substantially cylindrical rib or wall (12, 64, 145), the flange having a medical grade adhesive pad attached thereto, the rib having a flexible and deflectable seal strip (14, 68, 148), located radially inwardly thereof, the bag side coupling element having a base wall (30, 83, 96) attached to a substantially annular wall (32, 64, 94) positioned for engagement with, and so as to be encircled by, the seal strip (14, 68, 148) on the body side coupling element, the bag side coupling element also having an annular outer wall (36, 82, 92) of resilient stretchable synthetic plastics material which encircles the base wall (30, 83, 96) and is joined to the base wall by an integral annular plastics hinge (38, 85, 95), whereby in use the said outer wall (36, 82, 92) acts as a flip-over ring and can be snapped between two limiting positions by direct finger action, the wall in its first or open position extending generally outwardly of the base wall (30, 83, 96) and in its second or closed position being located adjacent to both the flange (10, 66, 142) and the wall (12, 64, 145) of the body side coupling element.

2. An ostomy coupling according to claim 1 in which there is at least one gripping tab (49A, 49B) on the bag side coupling element.

3. An ostomy coupling according to claim 1 or 2 in which construction is such that there is a detent or locking relationship existing between an overhang (18) on the radially outer rim of the substantially cylindrical rib and a step (48) on the radially inner surface of the outer wall of the bag side coupling element.

4. An ostomy coupling according to any of claims 1 to 3 and of which one coupling element is ring-like and has a channel shape in cross section, defined by radially inner and outer walls and a floor, the element being characterised in that (i) the floor of the element has a plurality of depressions therein adjacent to the inner wall, the depressions being equally spaced around the channel, and (ii) the radially outer wall has a plurality of inwardly-projecting shelf-like roof portions, equally spaced around the channel and extending partly across the channel from that part of the outer wall furthest from the floor of said channel.

5. A bag side coupling element for use in an ostomy coupling, the element comprising a base wall (30) attached to a substantially annular wall (32), and an annular outer wall (36) of resilient stretchable synthetic plastics material which encircles the base wall and is joined to the base wall by an integral annular plastics hinge (38) whereby in use the said outer wall can be snapped between two limiting positions.

## Patentansprüche

1. Stomakupplung mit einem körperseitigen Kupplungselement mit einem Flansch (10, 66, 142), von dem sich eine im wesentlichen zylindrische Rippe oder Wand (12, 64, 145) erstreckt, wobei an dem Flansch eine medizinische Heftkompresse befestigt ist, die Rippe einen flexiblen und ablenkharen Dichtungsstreifen (14, 68, 148) hat, der radial einwärts von ihr angeordnet ist, das beutelseitige Kupplungselement eine Basiswand (30, 83, 96) hat, die an einer im wesentlichen ringförmigen Wand (32, 64, 94) befestigt ist, die zum Eingriff mit dem Dichtungsstreifen (14, 68, 148) an dem körperseitigen Kupplungselement so positioniert ist, daß sie von ihm umgeben ist, das beutelseitige Kupplungselement ebenfalls eine ringförmige Außenwand (36, 82, 92) aus elastischem dehnbarem Kunststoffmaterial hat, die die Basiswand (30, 83, 96) umgibt und mit der Basiswand durch ein einstückiges ringförmiges Kunststoffscharnier (38, 85, 95) verbunden ist, wodurch im Gebrauch die Außenwand (36, 82, 92) als Umklappring wirkt und zwischen zwei Grenzpositionen durch direkte Fingerwirkung umgeschlagen werden kann, wobei sich die Wand in ihrer ersten oder offenen Position allgemein auswärts von der Basiswand (30, 83, 96) erstreckt und in ihrer zweiten oder geschlossenen Position benachbart sowohl zu dem Flansch (10, 66, 142) als auch der Wand (12, 64, 145) des körperseitigen Kupplungselements angeordnet ist.

2. Stomakupplung nach Anspruch 1, wobei mindestens eine Greiflasche (49A, 49B) an dem beutelseitigen Kupplungselement vorhanden ist.

3. Stomakupplung nach Anspruch 1 oder 2, wobei der Aufbau so ist, daß eine Arretier- oder Verschlußbeziehung vorhanden ist, die zwischen einem Überhang (18) an dem radialen Außenrand der im wesentlichen zylindrischen Rippe und einer Stufe (48) an der radialen Innenfläche der Außenwand des beutelseitigen Kupplungselements vorliegt.

4. Stomakupplung nach einem der Ansprüche 1 bis 3, wobei ein Kupplungselement ringartig ist und eine Kanalform im Querschnitt hat, die durch eine radiale Innen- und Außenwand sowie einen Boden gebildet ist, wobei das Element dadurch gekennzeichnet ist, daß (i) der Boden des Elements mehrere Vertiefungen hat, die zu der Innenwand benachbart sind, wobei die Vertiefungen gleichmäßig um den Kanal beabstandet sind, und (ii) die radiale Außenwand mehrere einwärts vorragende regalbrettartige Dachabschnitte hat, die gleichmäßig um den Kanal beabstandet sind und sich teilweise über den Kanal von jenem Teil der Außenwand erstrecken, der vom Kanalboden am weitesten entfernt ist.

5. Beutelseitiges Kupplungselement zur Verwendung in einer Stomakupplung, wobei das Element aufweist: eine Basiswand (30), die an einer im wesentlichen ringförmigen Wand (32) befestigt ist, und eine ringförmige Außenwand (36) aus elastischem dehnbarem Kunststoffmaterial, die die Basiswand umgibt und mit der Basiswand durch ein einstückiges ringförmiges Kunststoffscharnier (38) verbunden ist, wodurch im Gebrauch die Außenwand zwischen zwei Grenzpositionen umgeschlagen werden kann.

## Revendications

1. Raccord de stomie qui comporte un élément de raccord côté corps comprenant un rebord (10,66,142) d'où part une nervure ou paroi sensiblement cylindrique (12, 64, 145), ce rebord ayant un tampon adhésif de qualité médicale qui lui est fixé, la nervure comportant une bande d'étanchéité (14,68,148) souple et flexible, située radialement à l'intérieur de la nervure, l'élément de raccord côté poche comportant une paroi de base (30, 83, 96) fixée à une paroi sensiblement annulaire (32, 64, 94) disposée de façon à s'engager dans et à être entourée par la bande d'étanchéité (14, 68, 148) sur l'élément de raccord côté corps, l'élément de raccord côté poche comportant aussi une paroi extérieure annulaire (36, 82, 92) en matière plastique synthétique extensible et élastique, qui entoure la paroi de base (30, 83, 96) et est réunie à la paroi de base par une charnière annulaire en matière plastique (38, 85, 95) qui fait corps avec elle, de telle sorte que, à l'utilisation, ladite paroi extérieure (36, 82, 92) joue le rôle d'une bague à bascule et qu'on peut la faire basculer directement avec le doigt entre deux positions-limites, cette paroi, dans sa première position ou position ouverte, s'étendant généralement à l'extérieur de la paroi de base (30, 83, 96) et, dans sa seconde position ou position fermée, étant contiguë au rebord (10, 66, 142) et à la paroi (12, 64, 145) de l'élément de raccord côté corps.

2. Raccord de stomie selon la revendication 1, dans lequel il est prévu au moins une patte de préhension (49A, 49B) sur l'élément de raccord côté poche.

3. Raccord de stomie selon la revendication 1 ou 2, dans lequel, par construction, il existe une relation de détente ou de verrouillage entre un surplomb (18), sur le bord radialement extérieur de la nervure sensiblement cylindrique, et un décrochement (48) sur la surface radialement intérieure de la paroi extérieure de l'élément de raccord côté poche.

4. Raccord de stomie selon l'une quelconque des revendications 1 à 3 et dont l'un des éléments de raccord est en forme de bague et a en coupe la forme d'un U, délimité par des parois radialement intérieure et extérieure et par un fond, cet élément étant caractérisé en ce que (i) le fond de l'élément comporte plusieurs creux à proximité de la paroi intérieure, ces creux étant régulièrement répartis autour du U, et (ii) la paroi radialement extérieure comporte plusieurs parties couvrantes en forme de tablettes faisant saillie vers l'intérieur, régulièrement réparties autour du U et traversant partiellement le U depuis la partie de la paroi extérieure qui est la plus éloignée du fond dudit U.

5. Elément de raccord côté poche destiné à un raccord de stomie, cet élément comprenant une paroi de base (30) fixée à une paroi sensiblement annulaire (32), en matière plastique synthétique extensible et élastique, qui entoure la paroi de base et est réunie à la paroi de base par une charnière annulaire (38) en matière plastique, qui fait corps avec elle, de telle sorte que, à l'utilisation, on peut faire basculer ladite paroi extérieure entre deux positions-limites.
